# EUROPÄISCHE PATENTSCHRIFT

(11) **EP 0 491 981 B1**
(45) Veröffentlichungstag und Bekanntmachung des Hinweises auf die Patenterteilung: **15.02.1995**
(21) Anmeldenummer: 90125390.6
(22) Anmeldetag: 23.12.1990
(51) Int. Cl.: A61M 1/16, B01F 5/10

(54) **Automatische Anlage zur Herstellung von Konzentraten durch Mischung von Flüssigkeit mit löslichem Feststoff**
Automatic concentrate production device mixing a liquid and a soluble solid
Dispositif automatique de préparation de concentrés en mélangeant un liquide avec un solide soluble

(43) Veröffentlichungstag der Anmeldung: 01.07.1992
(73) Patentinhaber: Fresenius AG, 61350 Bad Homburg (DE)
(72) Erfinder: Polaschegg, Hans-Dietrich, Dr., W-6370 Oberursel 4 (DE); Steinbach, Bernd, Dr.-Ing., W-6380 Bad Homburg (DE); Walter, Claus, Dipl.-Ing., W-6380 Bad Homburg (DE)
(74) Vertreter: Fuchs Mehler Weiss

(56) Entgegenhaltungen:
- CH-A- 370 057
- DE-A- 3 844 174
- FR-A- 1 333 222
- FR-A- 2 339 431
- FR-A- 2 502 960
- US-A- 4 734 198

## Beschreibung

### Stand der Technik bei Konzentratherstellanlagen:

Aus der **OS-DE 3844174 A1** ist bereits eine ähnliche Anlage bekannt, bei welcher der aufzulösende Feststoff mit der erforderlichen Flüssigkeitsmenge in einen gemeinsamen Behälter gegeben und mittels einer Pumpe zur Auflösung gebracht wird. Gegenüber der vorliegenden Erfindung liegen die Nachteile dieser Anlage darin, daß die Pulverzugabe über eine hoch liegende Öffnung im Deckel des Mischbehälters vorgenommen werden muß. Desweiteren verfügt diese Anlage über eine Einrichtung zur Begasung. Der hohe Gasverbrauch dieser Einrichrung und die damit verbundenen hohen Kosten, beispielsweise bei der Anwendung des Gases CO₂, wird mit der erfindungsgemäßen Anlage **1** deutlich reduziert. Im Verfahren besteht ein weiterer Unterschied darin, daß mit der erfindungsgemäßen Anlage **1** Konzentrate mit unterschiediicher Konzentration hergestellt werden können und restliches Konzentrat, durch die Möglichkeit der Zusatzproduktion, nicht verworfen werden muß.

Ähnlich arbeitet auch die Konzentratherstellungsanlage der Firma Medizin-Technik-Walther GmbH in Ubstadt-Weiher, welche sich gegenüber der erfindungsgemäßen Anlage **1** in der Verfahrensführung grundlegend unterscheidet, was z.B. dadurch ersichtlich ist, daß für den Betrieb der Anlage zwei Pumpen erforderlich sind. Sie verfügt über keine Begasungseinheit bzw. es ist keine Anschlußmöglichkeit für ein Gas vorhanden. Auch bei dieser Anlage muß der aufzulösende Feststoff direkt in den Mischbehälter gegeben werden, dessen Öffnung, je nach Ausführung, ebenfalls sehr hoch liegt. Ein weiterer Nachteil gegenüber der erfindungsgemäßen Anlage **1** ist, daß sie, sofern sie für die Herstellung von Bicarbonatkonzentrat eingesetzt, über keine Filtrationseinheit verfügt.

Desweiteren sind Anlagen bekannt, in welchen die Auflösung des löslichen Feststoffes in Flüssigkeit und Mischung mittels eines Rührers vorgenommen wird, wie beispielsweise das RS-2500/RS-2130 Mischsystem der Firma Renal Systems (USA). Da gerade diese Anlage auch für die Herstellung von Bicarbonatkonzentrat konfiguriert ist, ist das Fehlen einer Filtrationseinheit und Begasungseinheit auch hier nachteilig aufzuführen. Ferner wird für das Entleeren der Anlage eine zusätzliche Pumpeinheit benötigt. Eine vollständige Entleerung ist nur durch nachteiliges Auskippen möglich und die Leitfähigkeitsmeßeinrichtung ist nicht fest installiert, weshalb eine sichere Qualitätskontrolle nicht sichergestellt ist. Letzlich wird auch hier der Feststoff direkt in den Auflösebehälter gegeben, dessen Füllöffnung sich nicht in einer ergonomischen Höhe befindet, wie sie der Vorlagebehälter **5** in der erfindungsgemäße Anlage **1** aufweist. Nach diesem Verfahren arbeitet auch die, in der **OS-DE 3443911 A1** beechriebene, Anlage. Wiederum muß der aufzulösende Feststoff über eine Öffnung in der Behälteroberseite zugegeben werden und das Konzentrat über eine zusätzliche Pumpe aus dem Behälter gefördert werden. Auch diese Anlage verfügt über keine Filtrationseinheit, wie sie beispielsweise für die Herstellung von Bicarbonatkonzentrat erforderlich ist. Eine Gasrezirkulation und eine Zugabemöglichkeit für Gas bzw. eine Begasungseinheit ist ebenfalls nicht implementiert.

Gegenüber der erfindungsgemäßen Anlage **1** haben alle aufgeführten Anlagen den Nachteil, daß die für die Desinfektion notwendige Zugabe von Desinfektionsmittel über die sehr ungünstig angebrachten Befüllöffnungen vorgenommen werden muß, welches eine gewisse Gefährdung für den Bediener mit sich bringt.

### Beschreibung:

Die Erfindung betrifft eine rechnergesteuerte Anlage zur Herstellung von Konzentraten durch Mischung von Flüssigkeit mit löslichem Feststoff, nach dem Oberbegriff des Anspruchs 1.

Insbesondere für die Herstellung von Bicarbonatkonzentrat zur Verwendung in Dialysegeräten besteht ein Bedürfnis nach einer automatischen Herstellungsanlage, welche die Produktion eines Konzentrates mit hoher Qualität ermöglicht, da vor allem in diesem Bereich höchste Anforderungen im Hinblick auf Sterilität und ausgewogene Konzentration bestehen.

Es ist daher Aufgabe der vorliegenden Erfindung, eine Anlage zur Herstellung von Konzentraten durch Mischen von Flüssigkeit mit löslichem Feststoff mit hohem Automatisierungsgrad zu schaffen, die eine gleichbleibend hoch qualitative Produktion von Konzentraten mit relativ niedrigem Aufwand ermöglicht.

Die Lösung der Aufgabe erfolgt durch die Merkmale im Anspruch 1.

Dadurch wird es ermöglicht, daß beispielsweise für die Herstellung von Dialysekonzentrat Bicarbonatpulver oder Granulat mit RO-Wasser (Umkehrosmosewasser) zur Auflösung gebracht werden kann, wobei durch die Zugabe von CO₂ das Konzentrat im pH-Wert ausgeglichen und danach vorzugsweise gefiltert werden kann, um die erforderliche Sterilität sicherzustellen.

Zu den besonderen Vorteilen der erfindungsgemäßen Anlage gehört der hohe Automatisierungsgrad, der integrierte und voll automatische Systemtest, die verbrauchsoptimierte Begasung, die einfache Handhabung und ihre kosteneffektive Produktionsweise, bei der Konzentrate mit hoher Qualität hergestellt werden können. Handelt es sich um Dialysekonzentrate, wie beispielsweise ein Bikarbonatkonzentrat, kann dessen pH-Wert optimal eingestellt werden, um eine Carbonatausfällung im Dialysegerät zu verhindern. Darüber hinaus ist es möglich, eine Kontamination mit Bakterien oder Pyrogenen wirksam zu verhindern bzw. auf das maximal zulässige Maß abzusenken.

Darüber hinaus können die Abmessungen der Anlage klein gehalten werden, so daß sie sich insbesondere als Kleinanlage im Bereich der Dialysetechnik hervorragend eignet. Ferner ist es möglich, die Anlage beweglich auszubilden, wozu sie in einem entsprechend ausgebildeten Gestell angeordnet werden kann, das beispielsweise durch das vorsehen von Rädern verfahrbar gemacht werden kann. Bei dieser Ausführung kann die Anlage, durch im Gestell angebrachte, justierbare Stützen, sicher aufgestellt werden.

Mit der erfindungsgemäßen Anlage **1** ist es möglich, für den Dialysebereich ein Bicabbonatkonzentrat, durch volumetrische Zugabe von geeignet aufbereiteten Wassers zu Bicarbonatpulver, anzusetzen, wobei eine vollständige Auflösung des aufzulösenden Feststoffes erreicht wird. Die Konzentration der Lösung wird beispielsweise über eine Leitfähigkeitsmessung gesteuert und der pH-Wert wird durch Einleiten von CO₂ Blasen in die Lösung eingestellt. Das Endprodukt wird vor dem Abfüllen in Flaschen oder Kanistern, bzw. vor Verteilung über Rohrleitungen zu den Dialysegeräten, gefiltert, um eventuell vorhandene Bakterien und Pyrogene zu eliminieren.

Die Unteransprüche haben vorteilhafte Weiterbildungen der Erfindung zum Inhalt.

Als eine besonders bevorzugte Filtrationseinheit kann beispielsweise ein PS 600 Membranfilter verwendet werden, wie er auch im Dialysierflüssigkeitsfilter der Fa. Fresenius AG zum Einsatz kommt.

Weitere Einzelheiten, Merkmale und Vorteile der Erfindung ergeben sich aus nachfolgender Beschreibung eines Ausführungsbeispiels anhand der Zeichnung.

Es zeigt:
**Fig.1** eine Schemazeichnung in Form eines Blockschaltbildes einer erfindungsgemäßen Anlage,
**Fig.2** eine Schemazeichnung in Form einer Schnittzeichnung des Deckel einer erfindungsgemäßen Anlage und
**Fig.3** eine Schemazeichnung der Abfülleinheit einer erfindungsgemäßen Anlage.

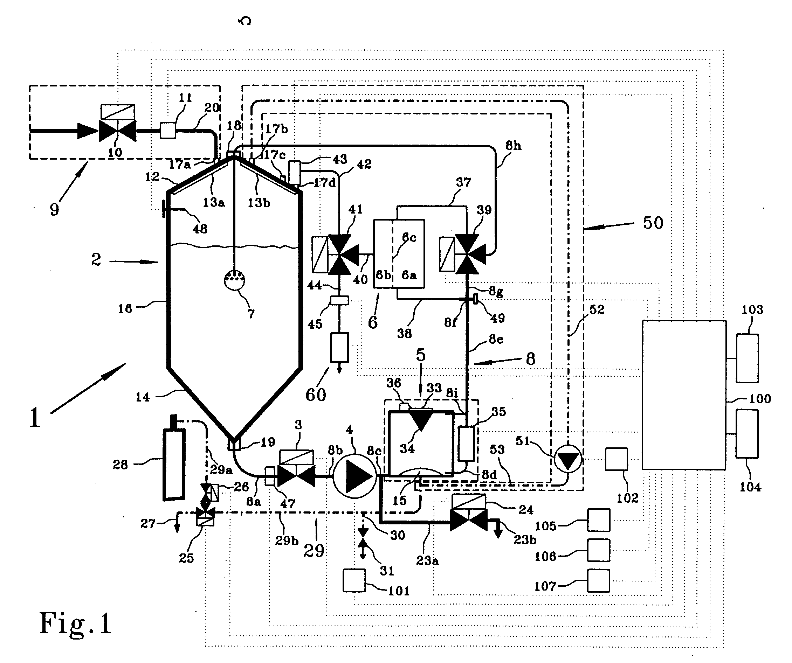

In **Fig.1** ist eine Ausführungsform einer erfindungsgemäßen Anlage **1** zur Herstellung von Konzentraten durch Mischung von Flüssigkeit mit löslichem Feststoff dargestellt. Eine derartige Anlage kann insbesondere zur Herstellung von Dialysekonzentraten als Kleinanlage ausgebildet sein, die in einem beispielsweise aus nichtrostendem Stahl hergestellten Rahmen montiert werden kann, der in **Fig.1** jedoch nicht näher dargestellt ist. Um eine derartige Anlage verfahrbar auszustatten, kann der Rahmen mit Rädern versehen werden, wobei hierfür die Anlagenstützen verstellbar ausgeführt sein müssen. Beispielsweise weist eine derartig aufgebaute Anlage Abmessungen auf, die im praktischem Betrieb ein Verfahren der gesamten Einheit durch Türen üblicher Abmessungen gestattet. Dementsprechend werden Abmessungen von ungefähr 1,98 m x 0.83 m bei eventuell abgehobenen Deckel nicht überschritten.

Die in **Fig.1** dargestellt Anlage weist im einzelnen eine Mischbehältereinheit **2** auf, die einen oberen zylindrischen Abschnitt **16** und einen trichterförmigen Boden **14** aufweist. Der Öffnungswinkel des trichterförmigen Bodens **14** beträgt vorzugsweise weniger als 80°.

Die Mischbehältereinheit **2** verfügt ferner über einen trichterförmigen Deckel **12**, welcher mit zwei sich gegenüber liegenden und zur Behälterinnenseite angeordneten Rinnen **13a** und **13b**, sowie mit einem Hauptzuführstutzen **18** ausgestattet ist und über vier Anschlußstutzen **17a**, **17b**, **17c** und **17d** verfügt. Der Öffnungswinkel des trichterförmigen Deckels **12** beträgt vorzugsweise mehr als 100°.

Desweiteren ist die Mischbehältereinheit **2** mit einem Überlaufdetektor **48** versehen, der beispielsweise als optischer Sensor ausgebildet sein kann.

Die Flüssigkeit, vorzugsweise RO-Wasser, wird aus einem nicht näher dargestellten Flüssigkeitsspeicher über den Flüssigkeitszulauf **9**, bestehend aus einem vorzugsweise elektrisch betätigbaren Dosierorgan **10**, einer Durchflußmesseinheit **11** und einer schematisch dargestellten Förderleitung **20**, und den Anschlußstutzen **17a** im Deckel **12** in die Mischbehältereinheit **2** geleitet.

Die Mischbehältereinheit **2** weist ferner einen Auslaßstutzen **19** auf, der am tiefsten Punkt des trichterförmigen Bodens **14** mit dem Behälterinneren verbunden ist.

Ferner ist in **Fig.1** verdeutlicht, daß die erfindungsgemäße Anlage einen Hauptflüssigkeitskreislauf **8** aufweist, welcher zur weiteren Verdeutlichung in die Teilsegmente **8a**, **8b**, **8c, 8d**, **8e**, **8f**, **8g**, **8h** und **8i** aufgeteilt ist. Im Teilsegment **8a**, das die Mischbehältereinheit **2** über den Auslaßstutzen **19** mit dem Absperrorgan **3** verbindet, ist eine Druckmeßeinrichtung **47** zur Füllstandskontrolle angebracht. Zwischen den beiden Teilsegmenten **8a** und **8b** ist ein vorzugsweise elektrisch betätigbares Absperrorgan **3** angeordnet. Zwischen dem Teilsegment **8b** und **8c** des Hauptflüssigkeitskreislaufes **8** ist eine Umwälzpumpe **4** angeordnet, welche über das Teilsegment **8c** mit dem Vorlagebehälter **5** verbunden ist.

Vom Teilsegment **8c** zweigt eine Ablaufleitung **23a** ab. Sie führt über ein vorzugsweise elektrisch betätigbares Absperrorgan **24** und die Ablaufleitung **23b** in einen nicht näher dargestellt Ablauf.

Im Bodenteil des Vorlagebehälter **5** ist eine Begasungseinheit **15** angebracht, welche über die Leitung **29**, bestehend aus den Teilsegmenten **29a** und **29b**, mit einem vorzugsweise elektrisch betätigbaren Entlüftungsorgan **25** verbunden ist, das zum einen über ein vorzugsweise elektrisch betätigbares Dosierorgan **26** an eine Gasflasche **28** angeschlossen ist, worüber vorzugsweise Kohlendioxid (CO₂) in den Flüssigkeitskreislauf eingebracht werden kann, und zum anderen über die Leitung **27** eine Verbindung zur Umgebung aufweist, worüber eine Entlüftung vorgenommen werden kann. An das Leitungssegment **29b** ist desweiteren eine Leitung **30** angeschlossen, welche über ein Sicherheitsorgan **31** in die Umgebung führt.

Der Vorlagebehälter **5** weist ferner eine Öffnung **33** auf, dessen Verschlußeinsatz **34** die Form eines Kegels aufweist. Der Verschlußeinsatz **34** in der Öffnung **33** des Vorlagebehälters **5** ist mit einer Sicherungsklinke **36** versehen. Desweiteren verfügt der Vorlagebehälter **5** neben einem Einlaß **8c** über zwei Auslässe **8d** und **8i**, zwischen welchen eine Leitfähigkeits- und Temperaturmeßeinrichtung **35** angeordnet ist, und die mit dem Leitungssegment **8e** des Hauptflüssigkeitskreislaufes **8** verbunden sind.

Desweiteren ist im Hauptflüssigkeitskreislauf **8** zwischen den Teilsegmenten **8g** und **8h** ein vorzugsweise elektrisch betätigbares Schaltorgan **39** angeordnet. Vom Teilsegment **8g** zweigt eine Zweigleitung **38** ab, die eine Filtrationseinheit **6** aufweist. An der Kreuzstelle **8f** des Hauptflüssigkeitskreislaufes **8** ist eine Druckmesseinrichtung **49** angeordnet.

Die Filtrationseinheit **6** besteht vorzugsweise aus einem Kapillarfilter mit Ausschlußfähigkeit für Bakterien und Endotoxinen. Ferner ist sie mit einer Ableiteinrichtung **40** versehen, die nach Zahl der vorhandenen Filterelemente einen oder mehrere Anschlüsse und ein vorzugsweise elektrisch betätigbares Ventil **41** zur Entnahmemöglichkeit des hergestellten Konzentrates umfaßt.

Desweiteren ist eine Aufnahmeeinrichtung **43** für die Abfülleinheit **60** an dem Anschlußstutzen **17d** im Deckel **12** der Mischbehältereinheit **2** angeordnet, welche über die Bypass-Leitung **42** mit dem Auslaßschaltorgan **41** verbunden ist. Desweiteren ist die Aufnahmeeinrichtung **43** mit einem nicht näher beschriebenen Detektor versehen. Ferner führt vom Auslaßschaltorgan **41** eine Leitung **44**, welche vorzugsweise flexibel ausgeführt und in der eine Durchflußmeßeinheit **45** angebracht ist, zur Abfülleinheit **60**.

Von der Filtrationseinheit **6** aus verläuft eine Rückführleitung **37** zu dem vorzugsweise elektrisch betätigbaren Schaltorgan **39**.

Das Teilsegment **8h** des Hauptflüssigkeitskreislaufes **8** führt vom Schaltorgan **39** über den Hauptzuführstutzen **18** im Deckel **12** der Mischbehältereinheit **2** in den Innenraum der Mischbehältereinheit **2** und ist am Ende mit einem Sprühkopf **7** versehen, dessen Funktion später erläutert werden wird.

Wie **Fig.1** ferner verdeutlicht, weist die erfindungsgemäße Anlage **1** einen Gaskreislauf **50** auf. Die Gaspumpe **51** ist über die Leitung **52** und den Anschlußstutzen **17b** mit dem Deckel **12** der Mischbehältereinheit **2** verbunden. Über die Leitung **53** ist die Gaspumpe **51** an die Leitung **29** angeschlossen.

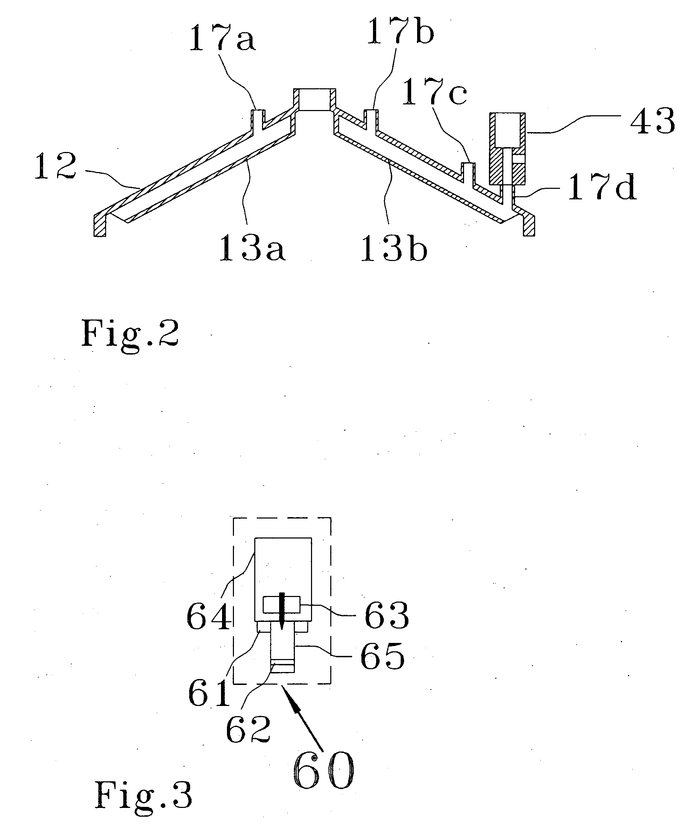

In der in **Fig.3** genauer dargestellten Abfülleinheit **60**, welche zur Kanister- und Kanisterfüllstandsdetektion dient, ist im Gehäuse **64** ein mechanisch/optischer Sensor **63** angeordnet. In dem Füllstutzen **65**, welcher fest mit dem Gehäuse **64** verbunden ist, ist ein Leitfähigkeitsdetektor, bestehend aus der Außenelektrode **62** und einer nicht näher dargestellten Innenelektrode, angeordnet. Wird beispielsweise in der Aufnahmeeinheit **43**, welche der Aufnahme der Abfülleinheit **60** dient, als Detektor ein Reed-Kontakteingesetzt, ist an dem Füllstutzen ein Magnet **61** angebracht.

Sämtliche Sensoren, wie die Durchflußmeßeinheiten **11** und **45**, die Druckmeßeinrichtungen **47** und **49**, der Überlaufdetektor **48**, die Detektoreneinheit **46** und der Detektor in der Aufnahmeeinrichtung **43** sind mit der elektronischen Steuer-, Regel-, Meß- und Rechnereinheit **100** über Steuerleitungen verbunden. Desweiteren sind alle Organe, wie die Absperrorgane **3** und **24**, das Schaltorgan **39**, das Auslaßschaltorgan **41**, die Dosierorgane **10** und **26**, sowie das Entlüftungsorgan **25** ebenfalls an die elektronische Steuer-, Regel-, Meß- und Rechnereinheit **100** angeschlossen. Ferner werden von der Steuer-, Regel-, Meß- und Rechnereinheit **100** über eine Convertereinheit **101** die Umwälzpumpe **4**, und über eine Schalteinheit **102** die Gaspumpe **51** betrieben.

An die Steuer-, Regel-, Meß- und Rechnereinheit **100** ist eine Eingabeeihheit **103** angeschlossen, über welche die Anlage **1** bedient wird.

Die Eingabeeinheit **103**, welche hier im Detail nicht näher beschrieben ist, verfügt über eine optische Ausgabekomponente, vorzugsweise ein Display, und einer mechanischen Eingabekomponente, vorzugsweise eine Tastatur. Hierüber können verschieden Betriebszustände der Anlage **1** eingestellt oder aber Informationen, wie beispielsweise die Qualitätsparameter, abgefragt werden. Desweiteren verfügt die Anlage **1** über eine Ausgabekomponente **104**, vorzugsweise ein Drucker, welche ebenfalls an der Steuer-, Regel-, Meß- und Rechnereinheit **100** angeschlossen ist und worüber eine detaillierte Herstellungsdokumentation ausgegeben werden kann.

Ferner sind an der Steuer-, Regel-, Meß- und Rechnereinheit **100** eine akustische Signaleinheit **106**, vorzugsweise ein Lautsprecher, eine optische Signaleinheit **105**, vorzugsweise eine, einer Ampel entsprechenden Lichteinheit und eine Datenleseeinheit **107**, welche beispielsweise ein Barcodeleser sein kann, angeschlossen.

### Funktionsweise:

### A: Konzentratherstellung:

### Beschickung der Anlage mit Flüssigkeit und Feststoff:

Zur Herstellung eines Konzentrates wird zunächst der Vorlagebehälter **5**, über die Öffnung **33**, mit der gewünschten Menge an löslichem Feststoff, wie beispielsweise Bicarbonatpulver, befüllt, nachdem man durch die Sicherungsklinke **36** den Verschlußeinsatz **34** entriegelt hat und der Vorlagebehälter **5** vom Bediener geöffnet wurde. Die Menge des einzugebenden Feststoffes, beispielsweise die Anzahl der verwendeten Säcke, wird manuell über die Eingabeeinheit **103** in die Steuer-, Regel-, Meß- und Rechnereinheit **100** eingegeben. Die flexible Eingabekonfiguration für die Menge an löslichen Feststoff erlaubt beliebige Feststoffmengen, bis zu einer anlagengemäßen, spezifischen Obermenge von vorzugsweise 50 kg Natriumbikarbonatpulver, zu verarbeiten. Die dabei verwendete Einheit, wie zum Beispiel Kilogramm, Mol oder Sack ist frei wählbar Der Vorlagebehälter **5** wird nach Abschluß der Beschickung mit Feststoff vom Bediener wieder verschlossen und der Verschlußeinsatz **34** durch die Sicherungsklinke **36** im Anschluß daran automatisch wieder verriegelt.

### Dichtigkeitsprüfung:

In der darauffolgende Phase des Herstellungsprozesses wird das System mittels eines Drucktests auf seine Funktionalität hin getestet, wobei insbesondere geprüft wird, ob der Deckel **34** dicht verschlossen ist. Mittels der Gaspumpe **51** wird Gas in den Vorlagebehälter **5** gepumpt, wodurch zwischen dem Absperrorgan **3** und dem Schaltorgan **39**, einschließlich dem Kompartiment **6a** der Filtrationseinheit **6**, ein Druck aufgebaut. Sofern die Membran **6c** in der Filtrationseinheit **6** dicht ist, wird der Prüfdruck erreicht, der vorzugsweise nicht unter 1 bar liegt. Über den zu beobachtenden Druckabfall wird sowohl die Dichtigkeit als auch der Zustand der Filtrationseinheit **6** beurteilt. Liegt der Druckabfall in dem fest vorgegebenen Zeitraum von einer Minute unter vorzugsweise 0.1 bar, ist das System hinreichend dicht.

### Zeitvorwahl:

Im Anschluß daran, kann mittels einer in **100** enthaltenen Zeitschalteinriohtung gewählt werden, ob der Misch- und Auflösungsprozeß sofort oder zu einem frei einzustellenden Zeitpunkt gestartet werden soll.

### Flüssigkeitszugabe:

Der Auflösungsprozeß beginnt in der Weise, daß im Folgenden die Mischbehältereinheit **2** über den Flüssigkeitszulauf **9** mit vorzugsweise RO-Wasser beschickt wird, dessen Endvolumen unter der theoretisch benötigten Flüssigkeitsmengeliegt, um während der anschließenden Auflösungsphase eine Überkonzentration des herzustellenden Konzentrates zu erreichen. Der zulaufende Volumenstrom an Flüssigkeit wird über die Durchflußmeßeinheit **11** permanent erfaßt und mittels der Druckmeßeinrichtung **47** kontrolliert.

### Auflösung des Feststoffes:

Die Auflösung des Feststoffs erfolgt dadurch, daß nach der Flüssigkeitszugabe mittels der Umwälzpumpe **4** die Flüssigkeit aus der Mischbehältereinheit **2** abgesaugt, durch den mit löslichem Feststoff beladenen Vorlagebehälter **5**, den Hauptflüssigkeitskreislauf **8** nebst Schaltorgan **39**, zurück in die Mischbehältereinheit **2** gepumpt wird. Wird beispielsweise Bicarbonatkonzentrat hergestellt, bei welchem während des Auflösens CO₂ aus der Lösung austritt, wird über den Gaskreislauf **50** mittels der Gaspumpe **51** das entweichende Gas über dem Flüssigkeitspegel in der Mischbehältereinheit **2** abgepumpt und dieses über die Leitungen **53** und **29** und der Begasungseinheit **15** dem Konzentrat durch einperlen wieder zugeführt. Um das chemische Gleichgewicht definiert von Na₂CO₃ nach NaHCO₃ zu verschieben, kann zusätzlich CO₂ mittels der Gasflasche **28** über die Leitung **29** dem System zugeführt werden.

### Feineinstellung der Konzentration:

Sobald während des Auflösevorgangs mittels der Leitfähigkeits- und Temperaturmeßeinrichtung **35** eine stabile Überkonzentration des Konzentrates festgestellt wird, wird von der Steuer-, Regel-, Meß- und Rechnereinheit **100** die fehlende Flüssigkeitsmenge berechnet und über den Flüssigkeitszulauf **9**, mittels der Durchflußmeßeinrichtung **11**, schrittweise zudosiert. Dabei wird die Qualität der Lösung ständig mit der Leitfähigkeits- und Temperaturmeßeinrichtung **35** von der Steuer-, Regel-, Meß- und Rechnereinheit **100** überwacht, wie auch das Flüssigkeitsvolumen über die Druckmeßeinheit **47** in der Mischbehältereinheit **2**. Die Leitfähigkeitsmessung ist dabei vorteilhafterweise temperaturkompensiert.

### Abfüllung des Produktes:

Zum Abfüllen des Konzentrates in Flaschen oder Kanister wird eine Abfülleinheit **60** verwendet. Diese befindet sich, wenn nicht abgefüllt wird, in der dafür konzipierten Aufnahmeeinrichtung **43**. Sie wird nun aus dieser Ruhestellung genommen und in die Öffnung des Kanisters gesteckt, dessen Öffungsradius vorzugsweise 34 mm betragen sollte. Durch das Hereinstecken der Abfülleinheit **60** in die Kanisteröffnung wird über den mechanisch/optischen Sensor **63** die Befüllung des Kanisters in Gang gesetzt. Erreicht der Füllstand im Kanister seinen Sollwert, wird dies in der Abfülleinheit **60** über den Leitfähigkeitsdetektor registriert und der Befüllvorgang sofort gestoppt. Die Abfülleinheit **60** wird nun in die Öffnung des nächsten zu befüllenden Kanisters gesteckt und der Vorgang wiederholt sich. Mittels des Schaltorgans **39** läßt sich der Flüssigkeitsstrom durch die Filtrationseinheit **6** leiten und von dort, in Abhängigkeit davon, ob gerade eine Abfüllung stattfindet oder nicht, zur Abfülleinheit **60** bzw. über die Bypass-Leitung **42** zurück in die Mischbehältereinheit **2** führen. Wird die Befüllung unterbrochen oder beendet, wird die Abfülleinheit **60** wieder in der Aufnahmeeinrichtung **43** fixiert. In Abhängigkeit der Drehzahl der Umwälzpumpe **4**, welche über die Steuer-, Regel-, Meß- und Rechnereinheit **100** stufenlos eingestellt werden kann, und der Stellung des Schaltorgans **39** läßt sich der Druck und damit verbunden die Produktfördermenge variieren. Die Sensorik in der Abfülleinheit **60** und in der Aufnahmeeinrichtung **43** läßt einen Betrieb der Anlage **1** nur zu, wenn sich die Abfülleinheit entweder in der vorgesehen Aufnahmeeinheit **43**, oder sich während der Abfüllphase in der Flaschen- bzw. Kanisteröffnung befindet. Somit weist die Anlage **1** vorteilhafterweise keinerlei Totwasserzonen auf.

### B: Desinfektion:

Die erfindungsgemäße Anlage **1** muß in regelmäßigen Zeitabständen desinfiziert werden. Bei einer Anlage für den Dialysebereich, beispielsweise nach einer Füllung in Intervallen von ein bis mehreren Tagen. Vor Beginn der Desinfektion wird noch vorhandenes Konzentrat aus der erfindungsgemäßen Anlage **1** vollautomatisch entfernt. Zum Desinfizieren der erfindungsgemäßen Anlage **1** wird diese mit beispielsweise 100 l RO-Wasser und einem zugeführten Desinfektionsmittel gefüllt. Ein derartiges Desinfektionsmittel kann beispielsweise Peressigsäure sein, wobei die Konzentration der Spüllösung 0.2% betragen sollte. Die Zugabe des Desinfektionsmittels geschieht in der Weise, daß nach der Befüllung der Anlage der Vorlagebehälter **5** geflutet wird und im Anschluß daran, durch Einleiten eines definierten Gasvolumens in den Vorlagebehälter **5**, mittels der Gaspumpe **51** über die Begasungseinheit **15**, ein dadurch ebenfalls definiertes Flüssigkeitsvolumen aus dem Vorlagebehälter **5** verdrängt wird. Das Absperrorgan **3** wird daraufhin sofort geschlossen und der Verschlußeinsatz **34** entriegelt, in dem die Sicherungsklinke **36** geöffnet wird. Vom Bediener wird nun der Vorlagebehälter **5** geöffnet und die entsprechende Menge an Desinfektionsmittel in diesen gegeben. Im Anschluß daran wird der Vorlagebehälter **5** wieder verschlossen und verriegelt. Nachdem das System auf seine Dichtigkeit hin überprüft wurde, wird die Desinfektionslösung vorzugsweise 30 Minuten lang zirkuliert, bei wechselnden Positionen der Stellorgane. Diese Zirkulation erfolgt ebenfalls durch die Pumpe **4**, wobei bei entsprechenden Schaltstellungen der Sprühkopf **7** ebenfalls mit Desinfektionslösung beschickt werden kann, was es ermöglicht, sämtliche Behälterinnenwände mit Desinfektionslösung zu benetzen, da der Sprühkopf **7** im oberen Bereich der Mischbehältereinheit **2** angeordnet ist. Darüber hinaus ermöglicht die Verwendung eines derartigen Sprühkopfes **7**, daß die Menge an erforderlicher Flüssigkeit zur Desinfektion minimiert werden kann. Nach der Desinfektion wird die Anlage **1** mehrere Male vollautomatisch gespült. Durch Prüfung mit Teststreifen kann sichergestellt werden, daß keine Desinfektionsmittelrückstände sich mehr in der Anlage befinden.

Die Desinfektion kann auch alternativ durch Heißwasser, vorzugsweise von mehr als 70°C, erfolgen. Dafür wird eine nicht näher beschriebene Heizeinrichtung nach dem Stand der Technik eingebaut. Selbstverständlich kann auch bei Verwendung von Desinfektionsmittel mit erhöhter Temperatur gearbeitet werden.

### C: Spülung:

Die erfindungsgemäße Anlage **1** kann auch nur gespült werden. Der Spülvorgang wird über die Eingabeeinheit **103** aktiviert. Wie beim zuvor beschriebenen Desinfektionsvorgang, wird auch hier die erfindungsgemäße Anlage **1** mit vorzugsweise 100 l RO-Wasser befüllt und dieses im Anschluß daran einige Minuten, bei wechselnden Positionen der Stellorgane, mittels der Pumpe **4** zirkuliert. Eventuell noch vorhandenes Konzentrat, welches mittels der Druckmeßeinrichtung **47** festgestellt werden kann, wird zuvor aus der erfindungsgemäßen Anlage **1** vollautomatisch entfernt, indem das Absperrorgan **24** geöffnet und die Flüssigkeit mittels der Umwälzpumpe **4** abgepumpt wird.

### D: Bedienungskomfort:

Vorteilhafterweise ist die erfindungsgemäße Anlage **1** äußerst einfach zu bedienen. Zunächst muß die Anlage **1** mit eine Energieversorgung verbunden werden und es muß sichergestellt sein, daß der Flüssigkeitszulauf, sowie und sofern gewünscht, die entsprechende Gasflasche **28**, angeschlossen sind. Dann kann die Anlage **1** mittels eines Einschaltorgans, beispielsweise einen an der Steuer-, Regel-, Meß- und Rechnereinheit **100** befindlichen Netzschalter, eingeschaltet werden. Danach ist die Anlage sofort betriebsbereit und der aktuelle Zustand ist über die Ausgabekomponente entnehmbar. Da die Anlage **1** über eine batteriegepufferte Speichereinheit verfügt, in welcher alle Anlagenparameter gespeichert sind, können vom System verschiedene Aufforderungen bzw. notwendige Maßnahmen dem Bediener mitgeteilt werden. So kann beispielsweise nach einem längeren Stillstand die Aufforderung zu einer Desinfektion erscheinen, die aus Sicherheitsgründen nicht umgangen werden kann. Verschiedene Prozesse, wie beispielsweise eine Spülung, können vom Bediener zu jeder Zeit und unabhängig vom aktuellen Zustand der Anlage **1** vorgenommen werden.

### E: Zentralversorgung:

Auch kann die erfindungsgemäße Anlage **1** als Zentralversorgungsanlage für zur Versorgung von mehreren Dialysegeräten mit einem Konzentrat, beispielsweise Bicarbonatkonzentrat, in Dialysestationen eingesetzt werden. Hierzu wird die erfindungsgemäße Anlage **1** über die Leitung **44**, in welcher die Durchflußmeßeinheit **45** angeordnet ist, direkt mit einem Versorguggsringleitungssystem bzw. einer Versorgungsstichleitungssystem verbunden, welches in **Fig.1** nicht näher dargestellt ist. Eine weitere, vorteilhafte Möglichkeit der Anbindung an ein zentrales Versorgungssystem kann in der Weise realisiert werden, daß die Abfülleinheit **60** mittels eines nicht näher dargestellten Gegenstückes an die Versorgungsleitung angeschlossen wird. Wird beispielsweise ein Versorgungsringleitungssystem angeschlossen, so kann dessen Rücklauf an dem Anschlußstutzen **17c** angebracht werden, wodurch vorteilhafterweise das rückgeführte Konzentrat in die Mischbehältereinheit **2** geleitet wird und somit erneut die Filtrationseinheit **6** passieren muß, bevor es erneut mittels der Umwälzpumpe **4** in die Versorgangsringleitung gepumpt wird.

Mit der erfindungsgemäßen Anlage **1** ist es möglich, Konzentrate mit höchster Qualität, automatisch und zeitgesteuert herzustellen und dabei einen einfachen und betriebssicheren Ablauf zu gewährleisten.
- 1: Anlage
- 2: Mischbehältereinheit
- 3: Absperrorgan
- 4: Umwälzpumpe
- 5: Vorlagebehälter
- 6: Filtrationseinheit
- 7: Sprühkopf
- 8: Hauptflüssigkeitskreislauf
- 8a: Leitungssegment
- 8b: Leitungssegment
- 8c: Leitungssegment/Einlaßleitung
- 8d: Leitungssegment/Auslaßleitung
- 8e: Leitungssegment
- 8f: Kreuzstück
- 8g: Leitungssegment
- 8h: Leitungssegment
- 8i: Leitungssegment/Auslaßleitung
- 9: Flüssigkeitszulauf
- 10: Dosierorgan
- 11: Durchflußmeßeinheit
- 12: Deckel
- 13a: Rinne
- 13b: Rinne
- 14: trichterförmiger Boden
- 15: Begasungseinheit
- 16: zylindrischer Abschnitt
- 17a: Anschlußstutzen
- 17b: Anschlußstutzen
- 17c: Anschlußstutzen
- 17d: Anschlußstutzen
- 18: Hauptzuführstutzen
- 19: Auslaßstutzen
- 20: Förderleitung
- 23a: Ablaufleitung
- 23b: Ablaufleitung
- 24: Absperrorgan
- 25: Entlüftungsorgan
- 26: Dosierorgan
- 27: Leitung
- 28: Gasflasche
- 29: Gasleitung
- 29a: Leitungssegment
- 29b: Leitungssegment
- 30: Leitung
- 31: Sicherheitsorgan
- 32: Leitung
- 33: Öffnung
- 34: Verschlußeinsatz
- 35: Leitfähigkeits- u. Temperaturmeßeinrichtung
- 36: Sicherungsklinke
- 37: Rückführleitung
- 38: Zweigleitung
- 39: Schaltorgan
- 36: Sicherungsklinke
- 40: Ableiteinrichtung
- 41: Ventil
- 42: Bypass-Leitung
- 43: Aufnahmeeinrichtung
- 44: Leitung
- 45: Durchflußmeßeinheit
- 47: Druckmeßeinrichtung
- 48: Überlaufdetektor
- 49: Druckmeßeinrichtung
- 50: Gaskreislauf
- 51: Gaspumpe
- 52: Leitung
- 53: Leitung
- 60: Abfülleinheit
- 61: Magnet
- 62: Außenelektrode
- 63: optisch/mechanischer Detektor
- 64: Gehäuse
- 65: Füllstutzen
- 100: Steuer-, Regel-, Meß- und Rechnereinheit
- 101: Convertereinheit
- 102: Schalteinheit
- 103: Eingabeeinheit
- 104: Ausgabekomponente
- 105: optische Signaleinheit
- 106: akustische Signaleinheit
- 107: Datenleseeinheit

## Patentansprüche

1. Anlage zur Herstellung von Konzentrat, insbesondere Hämodialysekonzentrat, aus Wasser und löslichem Feststoff mit einer Mischbehältereinheit **(2)**, einer Wasserzuführeinheit **(9)**, einem Flüssigkeitskreislauf **(8)**, einer Abfülleinheit **(60)** und einer Filtereinheit **(6)**, sowie einer Gaszuführeinrichtung **(25)** bis **(29)**, **dadurch gekennzeichnet,** daß in dem Flüssigkeitskreislauf **(8)** ein Vorlagebehälter **(5)** zur Aufnahme des Feststoffes eingebaut ist.

2. Anlage nach Anspruch 1, dadurch gekennzeichnet, daß der trichterförmige Boden der Mischbehältereinheit **(2)** einen Öffnungswinkel kleiner 80° aufweist.

3. Anlage nach Anspruch 1, dadurch gekennzeichnet, daß der Vorlagebehälter **(5)** eine Begasungseinheit **(15)** im Behälterboden aufweist.

4. Anlage nach Anspruch 1, dadurch gekennzeichnet, daß der Vorlagebehälter **(5)** einen kegelförmigen Verschlußeinsatz **(34)** aufweist.

5. Anlage nach Anspruch 4, dadurch gekennzeichnet, daß der Verschlußeinsatz **(34)** durch einer Sicherungsklinke **(36)** sicherbar ist. ist.

6. Anlage nach Anspruch 1, dadurch gekennzeichnet, daß der Vorlagebehälter **(5)**, eine tangential angeordneten Einlaßleitung **(8c)** und zwei ebenfalls tangential angeordneten Auslaßleitungen **(8d)** und **(8i)** aufweist.

7. Anlage nach Anspruch 6, dadurch gekennzeichnet, daß zwischen den beiden tangential angeordneten Auslaßleitungen **(8d)** und **(8i)** eine Leitfähigkeits/Temperatur-Meßeinheit **(35)** angeordnet ist.

8. Anlage nach Abspruch 1, dadurch gekennzeichnet, daß im Flüssigkeitszulauf **(9)** ein vorzugsweise elektrisch betriebenes Absperrventil **(10)** und eine Durchflußmeßeinrichtung **(11)** angeordnet ist.

9. Anlage nach Anspruch 1, dadurch gekennzeichnet, daß ein zusätzlicher Gaskreislauf **(50)** vorgesehen ist, der eine Gaspumpe **(51)** aufweist, die über die Leitung **(52)** an den Deckel **(12)** und über die Leitung **(53)** an die Begasungsleitung **(29)** angeschlossen ist.

10. Anlage nach Anspruch 1, dadurch gekennzeichnet, daß eine Abfülleinheit **(60)** vorgesehen ist, die einen mechanisch/optischen Detektor **(63)** aufweist, der ein Signal abgibt, wenn die Abfülleinheit **(60)** sich in einem geeigneten Kanister befindet, dessen Stutzen vorzugsweise einen Innendurchmesser von 34 mm bis 60 mm aufweist.

11. Anlage nach Anspruch 10, dadurch gekennzeichnet, daß die Abfülleinheit **(60)** einen Füllstandssensor **(62)** aufweist, der ein Signal abgibt, wenn der Sollfüllstand im Kanister erreicht ist.

## Claims

1. Plant for producing concentrate, in particular haemodialysis concentrate, from water and soluble solid using a mixing container unit (2), a water supply unit (9), a liquid circuit (8), a pouring unit (60) and a filter unit (6), and a gas supply device (25) to (29), characterised in that a collecting container (5) is installed in the liquid circuit (8) to receive the solid.

2. Plant according to claim 1, characterised in that the funnel-shaped base of the mixing container unit (2) has an angle of opening less than 80°.

3. Plant according to claim 1, characterised in that the collecting container (5) has a gassing unit (15) in the container base.

4. Plant according to claim 1, characterised in that the collecting container (5) has a cone-shaped closure insert (34).

5. Plant according to claim 4, characterised in that the closure insert (34) can be secured by means of a safety catch (36).

6. Plant according to claim 1, characterised in that the collecting container (5) has a tangentially arranged inlet pipe (8c) and two outlet pipes (8d) and (8i) likewise tangentially arranged.

7. Plant according to claim 6, characterised in that a conductivity/temperature measuring unit (35) is arranged between the two tangentially arranged outlet pipes (8d) and (8i).

8. Plant according to claim 1, characterised in that a preferably electrically operated shut-off valve (10) and a flow measuring device (11) is arranged in the liquid supply (9).

9. Plant according to claim 1, characterised in that an additional gas circuit (50) is provided, which has a gas pump (51) which is connected to the cover (12) via pipe (52) and to the gassing pipe (29) via pipe (53).

10. Plant according to claim 1, characterised in that a pouring unit (60) is provided, which has a mechanical/optical detector (63) which emits a signal if the pouring unit (60) is situated in a suitable canister, the connection of which preferably has an internal diameter of 34 mm to 60 mm.

11. Plant according to claim 10, characterised in that the pouring unit (60) has a level sensor (62) which emits a signal if the theoretical level in the canister is reached.

## Revendications

1. Installation pour la préparation d'un concentré, en particulier d'un concentré pour hémodialyse, à partir d'eau et d'une matière solide soluble, comportant une unité à malaxeur (2), une unité d'alimentation en eau (9), un circuit de liquide (8), une unité de remplissage (60) et une unité à filtre (6), ainsi qu'un dispositif d'arrivée de gaz (25 à 29), caractérisée en ce que dans le circuit de liquide (8) est monté un récipient (5) servant à recevoir la matière solide.

2. Installation selon la revendication 1, caractérisée en ce que le fond en forme d'entonnoir de l'unité à malaxeur (2) présente un angle d'ouverture inférieur à 80°.

3. Installation selon la revendication 1, caractérisée en ce que le récipient (5) comporte une unité (15) d'absorption du gaz, dans son fond.

4. Installation selon la revendication 1, caractérisée en ce que le récipient (5) comporte un insert de fermeture (34) conique.

5. Installation selon la revendication 4, caractérisée en ce que l'insert de fermeture (34) peut être bloqué au moyen d'un cliquet de sûreté (36).

6. Installation selon la revendication 1, caractérisée en ce que le récipient (5) comporte une conduite d'entrée (8c) placée tangentiellement et deux conduites de sortie (8d et 8i) également placées tangentiellement.

7. Installation selon la revendication 6, caractérisée en ce qu'entre les deux conduites de sortie (8d et 8i), placées tangentiellement, il est prévu une unité de mesure de conductibilité/température (35).

8. Installation selon la revendication 1, caractérisée en ce que dans l'arrivée de liquide (9) il est prévu une vanne d'arrêt (10), commandée de préférence électriquement, et un dispositif de mesure de débit (11).

9. Installation selon la revendication 1, caractérisée en ce qu'il est prévu un circuit de gaz (50) supplémentaire, qui comporte une pompe à gaz (51), qui est raccordée par la conduite (52) au couvercle (12) et par la conduite (53), à la conduite d'absorption de gaz (29).

10. Installation selon la revendication 1, caractérisée en ce qu'il est prévu une unité de remplissage (60), qui comporte un détecteur mécanique/optique (63), lequel délivre un signal lorsque l'unité de remplissage (60) se trouve dans un bidon approprié, dont la tubulure présente de préférence un diamètre inférieur compris entre 34 mm et 60 mm.

11. Installation selon la revendication 10, caractérisée en ce que l'unité de remplissage (60) comporte un capteur de niveau (62) qui délivre un signal, lorsque le niveau de consigne est atteint dans le bidon.
